# EUROPEAN PATENT APPLICATION

(11) **EP 2 792 746 A1**
(43) Date of publication of application: **22.10.2014**
(21) Application number: 12857414.2
(22) Date of filing: 12.12.2012
(51) Int. Cl.: C12N 15/113, A61K 31/711, A61K 48/00, A61P 3/06, C07H 21/04

(54) **OLIGONUCLEOTIDE AND THERAPEUTIC AGENT FOR HYPERLIPIDEMIA CONTAINING SAME AS ACTIVE INGREDIENT**

(30) Priority: 12.12.2011 JP 2011271751
(71) Applicant: National Cerebral and Cardiovascular Center, Suita-shi, Osaka 565-8565 (JP)
(72) Inventor: OBIKA, Satoshi, Suita-shi Osaka 565-0871 (JP); YAMAMOTO, Tsuyoshi, Suita-shi Osaka 565-0871 (JP); NAKATANI, Moeka, Suita-shi Osaka 565-0871 (JP); SHIBA, Mariko, Suita-shi Osaka 565-8565 (JP)
(74) Representative: Clements, Andrew Russell Niel
(86) International application number: PCT/JP2012/082255
(87) International publication number: WO 2013/089157

(57) **Abstract**

The oligonucleotide of the present invention includes a sugar-modified nucleoside, the sugar-modified nucleoside has a cross-linked structure between 4'-position and 2'-position, and the oligonucleotide is capable of binding to the apolipoprotein C-III gene. According to the present invention, an oligonucleotide useful as a therapeutic agent for hyperlipidemia that is excellent in binding affinity to the apolipoprotein C-III gene, stability and safety is provided.

## Description

### Technical Field

The present invention relates to an oligonucleotide and a therapeutic agent for hyperlipidemia containing the oligonucleotide as an active ingredient.

### Background Art

Hypertriglyceridemia in which a serum triglyceride (TG) level is increased is widely recognized as an independent risk of arteriosclerosis, and the importance of treatment for hypertriglyceridemia is beginning to be recognized in the prevention of coronary disease or cerebrovascular disease, after hypercholesterolemia in which a cholesterol (Chol) level is increased. Familial combined hyperlipidemia (FCHL) is a hereditary disease that accompanies hypertriglyceridemia and hypercholesterolemia, and it is reported that occurrence thereof is as high as 1 to 2% of the population and 20% of myocardial infarction patients aged less than 60 years suffer from FCHL. In the treatment for the concurrent disease of hypertriglyceridemia and hypercholesterolemia, the treatment for hypercholesterolemia in which a statin is mainly used is principally performed, and hypertriglyceridemia cannot be sufficiently treated in many cases because fibrate-based medicine tends to cause side effects when being used with statin.

Apolipoprotein C-III (ApoC3) constitutes high density lipoprotein (HDL) and TG-rich lipoprotein, and exhibits an action of increasing the serum TG level by inhibiting lipoprotein lipase (LPL) involved in hydrolysis of TG-rich lipoprotein. For example, since polymorphisms in the promoter region of the ApoC3 gene cause hypertriglyceridemia, hypocholesterolemia and hypotriglyceridemia occur in an ApoC3 knockout mouse and hypertriglyceridemia occurs in a transgenic mouse overexpressing ApoC3, it is suggested that ApoC3 can be a novel drug discovery target for hypertriglyceridemia.

Patent Document 1 reports a 2' MOE (2'-Omethoxyethyl) modified oligonucleotide targeting the ApoC3 gene. However, the modified oligonucleotide has excellent stability in vivo, but has low binding affinity to a target RNA as an antisense molecule, and therefore, there is a problem in that the modified oligonucleotide needs to be used in a very high dose to exhibit a medicinal effect.

Incidentally, Non-Patent Documents 1 to 9 and Patent Document 2 report a novel bridged artificial nucleic acid.

### Citation List

### Patent Documents

Patent Document 1: U.S. Patent No. 7,598,227
Patent Document 2: WO2011/052436

### Non-Patent Documents

Non-Patent Document 1: S. Obika et al, Tetrahedron Lett., 1997, Vol.38, pp.8735-8738
Non-Patent Document 2: S. Obika et al., Tetrahedron Lett., 1998, Vol.39, pp.5401-5404
Non-Patent Document 3: S. K. Singh et al, Chem. Commun., 1998, Vol.4, pp.455-456
Non-Patent Document 4: A. A. Koshkin et al., Tetrahedron, 1998, Vol.54, pp.3607-3630
Non-Patent Document 5: S. Obika et al., Bioorg. Med. Chem., 2001, Vol.9, pp.1001-1011
Non-Patent Document 6: S. M. A. Rahman et al., Angew. Chem. Int. Ed., 2007, Vol.46, pp.4306-4309
Non-Patent Document 7: S. M. A. Rahman et al., Nucleosides Nucleotides Nucleic Acids, 2007, Vol.26, pp.1625-1628
Non-Patent Document 8: K. Miyashita et al., Chem. Commun., 2007, Vol.36, pp.3765-3767
Non-Patent Document 9: S. M. A. Rahman et al., J. Am. Chem. Soc., 2008, Vol.130, pp.4886-4896

### Summary of the Invention

### Problem to be Solved by the Invention

It is an object of the present invention to provide an oligonucleotide useful as a therapeutic agent for hyperlipidemia that is excellent in binding affinity to the apolipoprotein C-III gene, stability and safety.

### Means for Solving the Problem

As a result of intensive research to solve the foregoing problems, the inventors of the present invention found that it is possible to provide an oligonucleotide useful as a therapeutic agent for hyperlipidemia that is excellent in binding affinity to the apolipoprotein C-III gene, stability and safety by including bridged cross-linked artificial nucleic acids in an oligonucleotide capable of binding to a specific target sequence of the apolipoprotein C-III gene, and the present invention was achieved.

The present invention provides an oligonucleotide comprising a sugar-modified nucleoside,
the sugar-modified nucleoside having a cross-linked structure between 4'-position and 2'-position, and
the oligonucleotide being capable of binding to an apolipoprotein C-III gene.

In one embodiment, the cross-linked structure is represented by -CO-NR¹-, -CH₂-CO-NR¹-, -(CH₂)₂-CO-NR¹-, -CO-NR¹-X-, or -CH₂-CO-NR¹-X-,
where R¹ represents a hydrogen atom;
an alkyl group having 1 to 7 carbon atoms that may be branched or form a cyclic group;
an alkenyl group having 2 to 7 carbon atoms that may be branched or form a cyclic group;
an aryl group having 3 to 12 carbon atoms that may have any one or more substituents selected from the group α consisting of a hydroxyl group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms and a halogen atom, and may contain a heteroatom; or
an aralkyl group with an aryl moiety having 3 to 12 carbon atoms that may have any one or more substituents selected from the group α and may contain a heteroatom; and
X represents an oxygen atom, a sulfur atom, an amino group or a methylene group.

In one embodiment, the cross-linked structure is represented by -CH₂-O-, -(CH₂)₂-O-, -CH₂-NR¹-O-, or -(CH₂)₂-NR¹-O-,
where R¹ represents a hydrogen atom;
an alkyl group having 1 to 7 carbon atoms that may be branched or form a cyclic group;
an alkenyl group having 2 to 7 carbon atoms that may be branched or form a cyclic group;
an aryl group having 3 to 12 carbon atoms that may have any one or more substituents selected from the group α consisting of a hydroxyl group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms and a halogen atom, and may contain a heteroatom; or
an aralkyl group with an aryl moiety having 3 to 12 carbon atoms that may have any one or more substituents selected from the group α and may contain a heteroatom.

In one embodiment, the apolipoprotein C-III gene is a human apolipoprotein C-III gene.

In one embodiment, the oligonucleotide is capable of binding to a 3'-untranslated region of the apolipoprotein C-III gene.

In one embodiment, a base sequence that becomes a basis for the oligonucleotide includes any one of the base sequences of Sequence ID Nos. 3 to 17, 20 to 24, and 26 to 53.

In one embodiment, the oligonucleotide has a base sequence length of 10 to 25 bases.

The present invention also provides a therapeutic agent for hyperlipidemia, comprising the above oligonucleotide as an active ingredient.

### Advantageous Effects of the Invention

According to the present study, an oligonucleotide useful as a therapeutic agent for hyperlipidemia can be provided that is excellent in binding affinity to the apolipoprotein C-III gene, stability and safety.

### Brief Description of Drawings

FIG. 1 is a graph illustrating ApoC3 mRNA expression levels of mouse NMuli cells treated with oligonucleotides.
FIG. 2 is a graph illustrating ApoC3 mRNA expression levels of human Huh-7 cells treated with oligonucleotides.
FIG. 3 is a graph illustrating a serum triglyceride level of high-fat diet-fed mice to which an oligonucleotide ApoC3-1-BNA or ApoC3-1-S was administered.
FIG. 4 is a graph illustrating an ApoC3 mRNA expression level in the liver of high-fat diet-fed mice to which an oligonucleotide ApoC3-1-BNA or ApoC3-1-S was administered.
FIG. 5 is a graph illustrating an ApoC3 protein expression level in the liver of high-fat diet-fed mice to which an oligonucleotide ApoC3-1-BNA or ApoC3-1-S was administered.
FIG. 6 is a graph illustrating a serum triglyceride level of high-fat diet-fed mice to which an oligonucleotide ApoC3-1-BNA (T, C) or ApoC3-1-NC (T, C) was administered.
FIG. 7 is a graph illustrating an ApoC3 mRNA expression level in the liver of high-fat diet-fed mice to which an oligonucleotide ApoC3-1-BNA (T, C) or ApoC3-1-NC (T, C) was administered.
FIG. 8 is a graph illustrating an ApoC3 protein expression level in the liver of high-fat diet-fed mice to which an oligonucleotide ApoC3-1-BNA (T, C) or ApoC3-1-NC (T, C) was administered.
FIG. 9 is a graph illustrating a serum triglyceride level of high-fat diet-fed mice to which an oligonucleotide ApoC3-2-NC was administered.
FIG. 10 is a graph illustrating an ApoC3 mRNA expression level in the liver of high-fat diet-fed mice to which an oligonucleotide ApoC3-2-NC was administered.
FIG. 11 is a graph illustrating an ApoC3 protein expression level in the liver of high-fat diet-fed mice to which an oligonucleotide ApoC3-2-NC was administered.
FIG. 12 is a graph illustrating a serum triglyceride level of high-fat diet-fed mice to which an oligonucleotide ApoC3-1-amide20 or ApoC3-1-amide 16 was administered.
FIG. 13 is a graph illustrating an ApoC3 mRNA expression level in the liver of high-fat diet-fed mice to which an oligonucleotide ApoC3-1-amide20 or ApoC3-1-amide 16 was administered.
FIG. 14 is a graph illustrating an ApoC3 protein expression level in the liver of high-fat diet-fed mice to which an oligonucleotide ApoC3-1-amide20 or ApoC3-1-amide16 was administered.
FIG. 15 is a graph illustrating changes over time in an ApoC3 mRNA expression level in the liver of high-fat diet-fed mice after a single intravenous administration of an oligonucleotide ApoC3-13-2-BNA was completed.
FIG. 16 is a graph illustrating changes over time in a serum triglyceride level of high-fat diet-fed mice after a single intravenous administration of an oligonucleotide ApoC3-13-2-BNA was completed.
FIG. 17 is a graph illustrating an ApoC3 mRNA expression level in the liver of high-fat diet-fed chronic hypertriglyceridemic mice after administrations of an oligonucleotide ApoC3-13-2-BNA was completed.
FIG. 18 is a graph illustrating changes over time in a serum triglyceride level of high-fat diet-fed chronic hypertriglyceridemic mice after administrations of an oligonucleotide ApoC3-13-2-BNA was started.
FIG. 19 is a graph illustrating a concentration of serum adiponectin in high-fat diet-fed chronic hypertriglyceridemic mice after the administrations of an oligonucleotide ApoC3-13-2-BNA was completed.
FIG. 20 is a graph illustrating serum transaminase levels of normal diet-fed mice to which various oligonucleotides were administered once intravenously.
FIG. 21 is a graph illustrating ApoC3 mRNA expression levels in the liver of normal diet-fed mice to which various oligonucleotides were administered once intravenously.
FIG. 22 is a graph illustrating amounts of nucleic acid of oligonucleotides accumulated in the liver of normal diet-fed mice to which various oligonucleotides were administered once intravenously.
FIG. 23 is a graph illustrating ApoC3 mRNA expression levels of mouse NMuli cells treated with various oligonucleotides.

### Description of Embodiments

First, the following definitions shall apply throughout the specification.

The term "linear alkyl group having 1 to 6 carbon atoms", as used herein, refers to any linear alkyl group having 1 to 6 carbon atoms and specifically a methyl group, an ethyl group, an n-propyl group, an n-butyl group, an n-pentyl group or an n-hexyl group.

The term "linear alkoxy group having 1 to 6 carbon atoms", as used herein, encompasses alkoxy groups having any linear alkyl group having 1 to 6 carbon atoms. Examples of the linear alkoxy groups include a methyloxy group, an ethyloxy group, an n-propyloxy group and the like.

The term "linear alkylthio group having 1 to 6 carbon atoms", as used herein, encompasses alkylthio groups with any linear alkyl group having 1 to 6 carbon atoms. Examples of the linear alkylthio groups include a methylthio group, an ethylthio group, an n-propylthio group and the like.

The term "linear alkylamino group having 1 to 6 carbon atoms", as used herein, encompasses alkylamino groups that have one or two amino groups having any one linear alkyl group having 1 to 6 carbon atoms or any two identical or different linear alkyl groups having 1 to 6 carbon atoms. Examples of the linear alkylamino group having 1 to 6 carbon atoms include a methylamino group, a dimethylamino group, an ethylamino group, a methylethylamino group and a diethylamino group.

The term "alkyl group having 1 to 7 carbon atoms that may be branched or form a cyclic group", as used herein, encompasses any linear alkyl groups having 1 to 7 carbon atoms, any branched alkyl groups having 3 to 7 carbon atoms having identical or different branched chains, any cyclic alkyl groups having 3 to 7 carbon atoms and a combination thereof having 4 to 7 carbon atoms. It may be simply referred to as "lower alkyl group". Examples of the any linear alkyl group having 1 to 7 carbon atoms include a methyl group, an ethyl group, a n-propyl group, a n-butyl group, a n-pentyl group, a n-hexyl group and a n-heptyl group. Examples of the any branched alkyl group having 3 to 7 carbon atoms having identical or different branched chains include an isopropyl group, an isobutyl group, a tert-butyl group and an isopentyl group. Examples of the any cyclic alkyl group having 3 to 7 carbon atoms include a cyclobutyl group, a cyclopentyl group and a cyclohexyl group.

The term "alkenyl group having 2 to 7 carbon atoms that may be branched or form a cyclic group ", as used herein, encompasses any linear alkenyl groups having 2 to 7 carbon atoms, any branched alkenyl groups having 2 to 7 carbon atoms, any cyclic alkenyl groups having 3 to 7 carbon atoms and a combination thereof having 4 to 7 carbon atoms. It may be simply referred to as "lower alkenyl group". Examples of the any linear alkenyl group having 2 to 7 carbon atoms include an ethenyl group, a 1-propenyl group, a 2-propenyl group, a 1-butenyl group, a 2-butenyl group, a 1-pentenyl group, a 2-pentenyl group, a 3-pentenyl group, a 4-pentenyl group and a 1-hexenyl group. Examples of the any branched alkenyl groups having 3 to 7 carbon atoms include an isopropenyl group, a 1-methyl-1-propenyl group, a 1-methyl-2-propenyl group, a 2-methyl-1-propenyl group, a 2-methyl-2-propenyl group and a 1-methyl-2-butenyl group. Examples of the any cyclic alkenyl group having 3 to 7 carbon atoms include a cyclobutenyl group, a cyclopentenyl group and a cyclohexenyl group.

The term "aryl group having 3 to 12 carbon atoms that may contain a heteroatom", as used herein, encompasses any aromatic hydrocarbons having 6 to 12 carbon atoms composed of only hydrocarbons and any heteroaromatic groups obtained by replacing one or more carbon atoms included in any ring structure having 6 to 12 carbon atoms with identical or different heteroatoms (e.g., a nitrogen atom, an oxygen atom and a sulfur atom). Examples of the aromatic hydrocarbon having 6 to 12 carbon atoms including only hydrocarbons include a phenyl group, a naphthyl group, an indenyl group and an azulenyl group. Examples of the heteroaromatic group include a pyridyl group, a pyrrolyl group, a quinolyl group, an indolyl group, an imidazolyl group, a furyl group and a thienyl group.

The term "aralkyl group with an aryl moiety having 3 to 12 carbon atoms that may contain a heteroatom", as used herein, encompasses any aromatic hydrocarbon compounds having 3 to 12 carbon atoms including only hydrocarbons and any heteroaromatic compounds obtained by replacing one or more carbon atoms included in any ring structure having 3 to 12 carbon atoms with identical or different heteroatoms (e.g., a nitrogen atom, an oxygen atom and a sulfur atom). Examples of the term "aralkyl group with an aryl moiety having 3 to 12 carbon atoms that may contain a heteroatom" include a benzyl group, a phenethyl group, a naphthylmethyl group, a 3-phenylpropyl group, a 2-phenylpropyl group, a 4-phenylbutyl group, a 2-phenylbutyl group, a pyridylmethyl group, an indolylmethyl group, a furylmethyl group, a thienylmethyl group, a pyrrolylmethyl group, a 2-pyridylethyl group, a 1-pyridylethyl group and a 3-thienylpropyl group.

Examples of the term "halogen atom", as used herein, include a fluorine atom, a chlorine atom, a bromine atom, and an iodine atom. A fluorine atom and chlorine atom are preferable.

The term "nucleoside", as used herein, refers to a glycosylamine that contains a nucleobase and a sugar. Examples of the nucleoside include, but are not limited to, naturally occurring nucleosides, abasic nucleosides, modified nucleosides and nucleosides having a pseudo base and/or sugar group.

The term "nucleotide", as used herein, refers to a glycosomine that contains a nucleobase and a sugar to which a phosphate group is covalently bonded. The nucleotide can be modified with any of various substituents.

The term "deoxyribonucleotide", as used herein, refers to a nucleotide that has a hydrogen atom at 2'-position of the sugar moiety of the nucleotide. The deoxyribonucleotide can be modified with any of various substituents.

The term "deoxyribonucleic acid (DNA)", as used herein, refers to a nucleic acid that contains a deoxyribonucleotide.

The term "ribonucleotide", as used herein, refers to a nucleotide that has a hydroxyl group at 2'-position of the sugar moiety of the nucleotide. The ribonucleotide can be modified with any of various substituents.

The term "ribonucleic acid (RNA)", as used herein, refers to a nucleic acid that contains a ribonucleotide.

The term "modified nucleoside", as used herein, refers to a non-naturally occurring type of the "nucleosides" in which a purine or pyrimidine base and a sugar are bonded and to a nucleoside in which a heteroaromatic ring or aromatic hydrocarbon ring that is neither a purine nor pyrimidine base and that can be used in place of a purine or pyrimidine base and a sugar are bonded. Preferable examples thereof include sugar-modified nucleosides in which the sugar moiety is modified.

The term "oligonucleotide", as used herein, refers to an "oligonucleotide" in which 2 to 50 identical or different "nucleosides" are bonded through a phosphodiester bond. It also includes a non-naturally occurring derivative of the "oligonucleotide". Preferable examples of such derivatives include sugar derivatives in which the sugar moiety is modified; thioate derivatives in which the phosphate diester moiety is thioated; phosphorothioate derivatives in which the oxygen atom of the phosphate group in the phosphodiester bond is replaced with a sulfur atom; esters in which the terminal phosphate moiety is esterified; and amides in which the amino group on the purine base is amidated, and more preferable examples thereof include sugar derivatives in which the sugar moiety is modified.

Hereinafter, the present invention will be described in detail.

The oligonucleotide of the present invention contains at least one sugar-modified nucleoside at any position. There is no particular limitation on the position and the number thereof, and the oligonucleotide may be designed as appropriate according to the purpose. Two or more sugar-modified nucleosides may be mutually the same or may be different.

The oligonucleotide of the present invention includes an oligonucleotide in which a naturally occurring DNA or RNA is chemically modified. Such modification changes the activity of the oligonucleotide. For example, it enhances affinity to the target nucleic acid, enhances resistance to a nucleolytic enzyme (nuclease), and changes the pharmacokinetics or histological distribution of the oligonucleotide. By enhancing the affinity of the oligonucleotide to the target, it can be possible to use a shorter oligonucleotide.

The oligonucleotide of the present invention can bind to apolipoprotein C-III (ApoC3).

Here, the term "can bind" means that a plurality of different single-strand oligonucleotides or nucleic acids can form a nucleic acid having two or more strands due to the complementarity of the nucleobase. Preferably, the term means that a double-strand nucleic acid can be formed. There is no particular limitation on the melting temperature (Tₘ) of the nucleic acid having two or more strands. For example, in two different single-strand oligonucleotides or nucleic acids that form a double-strand nucleic acid, the base sequences of the double-strand forming regions need not be completely complementary to each other.

The human ApoC3 gene includes the base sequence of Sequence ID No. 1 (GenBank accession number: NM_000040; 533 bases). The mouse ApoC3 gene includes the base sequence of Sequence ID No. 2 (GenBank accession number: NM_023114; 524 bases). The human ApoC3 is preferably used as the ApoC3.

In one embodiment, the oligonucleotide of the present invention is an oligonucleotide that can bind to a 3'-untranslated region (3' UTR) of the ApoC3 gene. The 3'-untranslated region corresponds to, for example, the region from positions 347 to 533 of Sequence ID No. 1 in a case of the human ApoC3 gene and the region from positions 353 to 524 of Sequence ID No. 2 in a case of the mouse ApoC3 gene.

In one embodiment, a base sequence that becomes a basis for the oligonucleotide of the present invention includes any one of the base sequences of Sequence ID Nos. 3 to 17, 20 to 24, and 26 to 53.

The term "a base sequence that becomes a basis" refers to a base sequence represented by replacing the bases in the sugar-modified nucleoside included in the oligonucleotide of the present invention with the native bases. For example, while the bases included in ApoC3-1-S that has the base sequence of Sequence ID No. 3 are native bases, ApoC3-1-BNA, ApoC3-1-BNA

(T, C), ApoC3-1-NC (T, C) and ApoC3-2-NC have the base sequences in which some bases of ApoC3-1-S are replaced with sugar-modified bases, that is, the base sequences that are based on Sequence ID No. 3.

The term "include any one of the base sequences of Sequence ID Nos. 3 to 17, 20 to 24, and 26 to 53" means that the base sequence represented by the Sequence ID No. may extend from its 5' end and/or 3' end as long as it can bind to a target sequence. It is possible to design such a base sequence and determine bases to be modified with sugar as appropriate based on the information of the gene sequence of the ApoC3 gene.

The sugar-modified nucleoside included in the oligonucleotide of the present invention has a cross-linked structure between 4'-position and 2'-position.

One of the above-described cross-linked structures is represented by - CO-NR¹-, -CH₂-CO-NR¹-, -(CH₂)₂-CO-NR¹-, -CO-NR¹-X-, or -CH₂-CO-NR¹-X-,
where R¹ represents a hydrogen atom;
an alkyl group having 1 to 7 carbon atoms that may be branched or form a cyclic group;
an alkenyl group having 2 to 7 carbon atoms that may be branched or form a cyclic group;
an aryl group having 3 to 12 carbon atoms that may have any one or more substituents selected from the group α consisting of a hydroxyl group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms and a halogen atom, and may contain a heteroatom; or
an aralkyl group with an aryl moiety having 3 to 12 carbon atoms that may have any one or more substituents selected from the group α and may contain a heteroatom; and
X represents an oxygen atom, a sulfur atom, an amino group or a methylene group. Hereinafter, such a cross-linked structure may be referred to as "AM".

Examples of AM include, but are not limited to, unsubstituted amide (4'-CO-NH-2'), N-methylamide (4'-CO-NCH₃-2'), N-isopropylamide (4'-CON(iPr)-2'), N-benzylamide (4'-CO-N(Bn)-2'), acetamide (4'-CH₂-CO-NH-2'), N-methylacetamide (4'-CH₂-CO-NCH₃-2'), N-oxyacetamide (4'-CH₂-CO-NH-O-2') and N-methyl-N-oxyacetamide (4'-CH₂-CO-NCH₃-O-2'). The AM nucleoside (monomer) or the oligonucleotide including it can be synthesized, for example, by a method described in Patent Document 2.

Another cross-linked structure described above is represented by - CH₂-O- or -(CH₂)₂-O-. Hereinafter, such a cross-linked structure may be referred to as "BNA".

Examples of BNA include, but are not limited to, α-L-methyleneoxy (4'-CH₂-O-2'), ß-D-methyleneoxy (4'-CH₂-O-2') and ethyleneoxy (4'-(CH₂)₂-O-2'). The BNA nucleoside (monomer) or the oligonucleotide including it can be synthesized, for example, by methods described in Non-Patent Documents 3 to 7.

Another cross-linked structure described above is represented by -CH₂-NR¹-O-, or -(CH₂)₂-NR¹-O-,
where R¹ represents a hydrogen atom;
an alkyl group having 1 to 7 carbon atoms that may be branched or form a cyclic group;
an alkenyl group having 2 to 7 carbon atoms that may be branched or form a cyclic group;
an aryl group having 3 to 12 carbon atoms that may have any one or more substituents selected from the group α consisting of a hydroxyl group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms and a halogen atom, and may contain a heteroatom; or
an aralkyl group with an aryl moiety having 3 to 12 carbon atoms that may have any one or more substituents selected from the group α and may contain a heteroatom. Hereinafter, such a cross-linked structure may be referred to as "NC".

Examples of NC include, but are not limited to, oxyamino (4'-CH₂-NH-O-2') and N-methyloxyamino (4'-CH₂-NCH₃-O-2'). The NC nucleoside (monomer) or the oligonucleotide including it can be synthesized, for example, by methods described in Non-Patent Documents 8 to 11.

There is no particular limitation on the base sequence length of the oligonucleotide of the present invention, but it is preferably 10 to 25 bases, and more preferably 13 to 20 bases.

Since the oligonucleotide including a sugar-modified nucleoside, or in particular, a sugar-modified nucleoside in which sugar modification is AM, is locked by the cross-linked structure, or in particular, a cross-linked structure containing an amide bond, between 4'-position and 2'-position as described above, the oligonucleotide is unlikely to be decomposed by various nucleases and can exist in a living body for a long period of time after being administered into the living body. For example, the oligonucleotide forms a stable double strand with mRNA and inhibits biosynthesis of pathogenic protein (an antisense method), or forms a triple strand with double-strand DNA in the genome and inhibits transcription to mRNA. Also, it becomes possible to suppress proliferation of a virus that has infected. Moreover, it is expected that the cross-linked structure containing an amide bond has high biocompatibility, and it can be further expected that the oligonucleotide also serves as an aptamer for recognizing a biogenic substance such as protein.

The ApoC3 gene exhibits an action of increasing a serum triglyceride (TG) level by inhibiting lipoprotein lipase (LPL) involved in hydrolysis of TG-rich lipoprotein. The oligonucleotide of the present invention can suppress the ApoC3 gene expression, for example, by binding to the mRNA of ApoC3 as an antisense nucleic acid. The LPL activity inhibited by ApoC3 is recovered by suppressing the ApoC3 gene expression, and the serum TG level can be reduced. Thus, the oligonucleotide of the present invention exhibits an effect as a therapeutic agent for hyperlipidemia.

The therapeutic agent for hyperlipidemia that contains the oligonucleotide of the present invention as an active ingredient may be blended with, for example, an auxiliary agent that is usually used in the technical field of pharmaceutical formulations, such as a vehicle, binder, preservative, oxidation stabilizer, disintegrator, lubricant, and corrigent, and be formulated into a parenteral preparation or liposomal preparation. Also, the therapeutic agent for hyperlipidemia may be blended with a pharmaceutical carrier that is usually used in this technical field and formulated into a topical preparation such as a solution, cream, and ointment.

### Examples

Hereinafter, the present invention will be described more specifically based on examples, but is not limited to the following examples.

### Example 1: Suppression of ApoC3 gene expression in vitro by oligonucleotide

### Selection of target region for oligonucleotide

The base sequence of the human ApoC3 gene (Sequence ID No. 1: 533 bases) was obtained from GenBank (accession number: NM_000040). The base sequence of the mouse ApoC3 gene (Sequence ID No. 2: 524 bases) was obtained from GenBank (accession number: NM_023114). These base sequences were analyzed by computer software to select the base sequence of a suitable region as a target for the oligonucleotide from the four viewpoints below:
(1) The folding structure of the mRNA for the ApoC3 gene was compared using mFold software (M. Zuker, Nucleic Acids Res., 2003, Vol. 31, pp. 3406-3415). A region in which a stem loop structure is unlikely to be formed on the mRNA was selected so that the oligonucleotide easily binds thereto.
(2) The base sequences of the human and mouse ApoC3 genes were compared using JustBio software (URL: http://www.justbio.com/). A region common in base sequences between human and mouse was selected so as to allow the application to human based on the results elevated for the mouse.
(3) A region with a large GC content was selected with a high heat stability when a double-strand nucleic acid was formed with the oligonucleotide.
(4) It was searched using Blast software (S. F. Altschul et al., J. Mol. Biol., 1990, Vol. 215, pp. 403-410) as to whether or not a base sequence similar to that of the target region was present in the remaining region of the genome. A region of a base sequence with low similarity to the base sequences of any other regions of the genome was selected so that the oligonucleotide does not bind to any other mRNAs.

The optimum target regions of the oligonucleotides were selected from the 4 conditions above to design respective oligonucleotides of base sequences complementary thereto (Table 1). It should be noted that, in Table 1, the term "PS backbone" refers to a structure in which the oxygen atom of the phosphate group in the phosphodiester bond is replaced with a sulfur atom (the group corresponding to the phosphate group is referred to as "phosphorothioate group"). Moreover, herein, an oligonucleotide in which all phosphate groups of the oligonucleotide are replaced with phosphorothioate groups is particularly referred to as "S-oligonucleotide". All the oligonucleotides in Table 1 are S-oligonucleotides. In Table 1, the target regions for the mouse ApoC3 gene (Sequence ID No. 2) are also shown. Moreover, the target regions for the oligonucleotides ApoC3-2-NC and ApoC3-2-BNA13 correspond to the regions from the positions 40 to 58 and from the positions 40 to 52 of the human ApoC3 gene (Sequence ID No. 1), respectively. The corresponding base sequences of the respective oligonucleotides are shown as Sequence ID Nos. 3 to 16 as shown in Table 1.

**Table 1**

| Oligonucleotide name | Base Sequence of Oligonucleotide | Target Region of Mouse ApoC3 Gene | Sequence ID No. |
|---|---|---|---|
| ApoC3-1-S | 5'-tcttatccagctttattagg-3' | 495-514 | 3 |
| ApoC3-1-BNA | 5'-TCtTaTCcagcttTaTTaGg-3' | | |
| ApoC3-1-BNA(T,C) | 5'-TCtTaTCcagcttTaTTagg-3' | | |
| ApoC3-1-NC(T,C) | 5'-*TC*t*T*a*TC*cagctt*T*a*TT*agg-3' | | |
| ApoC3-2-NC | 5'-*CC*gggg*CT*gcatgg*C*a*CCt*-3' | 46-64 | 4 |
| ApoC3-1-amide20 | 5'-tcTTaTccagcttTaTTagg-3' | 495-514 | 3 |
| ApoC3-1-amide16 | 5'-TTaTccagcttTaTTa-3' | 497-512 | 5 |
| ApoC3-1-BNA13 | 5'-ATCcagctttATT-3' | 498-510 | 6 |
| ApoC3-2-BNA13 | 5'-CTGcatggcaCCT-3' | 46-58 | 7 |
| ApoC3-3-BNA13 | 5'-CATggcacctACG-3' | 43-55 | 8 |
| ApoC3-4-BNA13 | 5'-TCGggcagatGCC-3' | 97-109 | 9 |
| ApoC3-5-BNA13 | 5'-TCCatgtagcCCT-3' | 150-162 | 10 |
| ApoC3-6-BNA13 | 5'-TCTtggaggcTTG-3' | 164-176 | 11 |
| ApoC3-7-BNA13 | 5'-GCTccagtagCCT-3' | 265-277 | 12 |
| ApoC3-8-BNA13 | 5'-TTAaagcaacCTT-3' | 424-436 | 13 |
| ApoC3-9-BNA13 | 5'-GCAgcttcttATC-3' | 508-520 | 14 |
| ApoC3-46-BNA14 | 5'-GCTgcatggcaCCt-3' | 46-59 | 15 |
| ApoC3-51-BNA14 | 5'-CCGgggctgcaTGg-3' | 51-64 | 16 |

| | | | |
|---|---|---|---|
| All are PS backbones. Underlined Capital Letter: AM, Italic Capital Letter: NC, Capital Letter: BNA, Small Letter: DNA AM represents 2',4'-BNA AM[N-R], wherein R=methyl. NC represents 2',4'-BNA _{NC}[N-R], wherein R=methyl. BNA represents 2',4'-BNA/LNA. | | | |

### Preparation of oligonucleotide

AM monomers (amidites) were synthesized by the method described in Patent Document 2. BNA monomers (amidites) were synthesized by the methods described in Non-Patent Documents 3 to 7. NC monomers (amidites) were synthesized by the methods described in Non-Patent Documents 8 to 11. Using these as a monomer for DNA synthesis, 1 to 100 mg (in vivo grade) of oligonucleotides were synthesized as appropriate with a DNA synthesizer, and were subjected to HPLC purification and lyophilization treatment. The purity and structure of each obtained oligonucleotide were confirmed with HPLC and MALDI-TOF-MS.

As shown in Table 1, the synthesized oligonucleotides (ApoC3 oligonucleotides) were S-oligonucleotides including no sugar-modified nucleoside (DNA-oligonucleotides: ApoC3-1-S and the like), oligonucleotides including a BNA-nucleoside (BNA-oligonucleotides: ApoC3-1-BNA and the like), oligonucleotides including an NC-nucleoside (NC-oligonucleotides: ApoC3-1-NC and the like), and oligonucleotides including an AM-nucleoside (AM-oligonucleotides: ApoC3-amide20 and the like).

### Oligonucleotide addition experiment on mouse hepatocyte

Mouse liver cell strain NMuli cells prepared so as to have 2.0×10⁵ cells/mL were seeded onto a 6-well plate in an amount of 2 mL per well, and cultured at 37°C under 5% CO₂ for 24 hours. In order to set a final concentration of the oligonucleotide to 50 nM, 110 µL of 1 µM oligonucleotide-containing solution, 14.3 µL of Lipofectamine 2000 (manufactured by Invitrogen) and 425.7 µL of Opti-MEM (manufactured by Invitrogen) were mixed, then the mixed solution was incubated at room temperature for 20 minutes, and 500 µL thereof and 1,500 µL of Opti-MEM were added to each well. The culture medium was replaced 4 hours after the addition of oligonucleotide. Cells were collected 20 more hours later and were disrupted with TRIzol Regent (manufactured by Invitrogen), and total RNA was extracted. The extracted total RNA was quantified with a spectrophotometer, and the length of the RNA was confirmed by agarose gel electrophoresis. Cells cultured in the medium to which no oligonucleotide was added were used as a control.

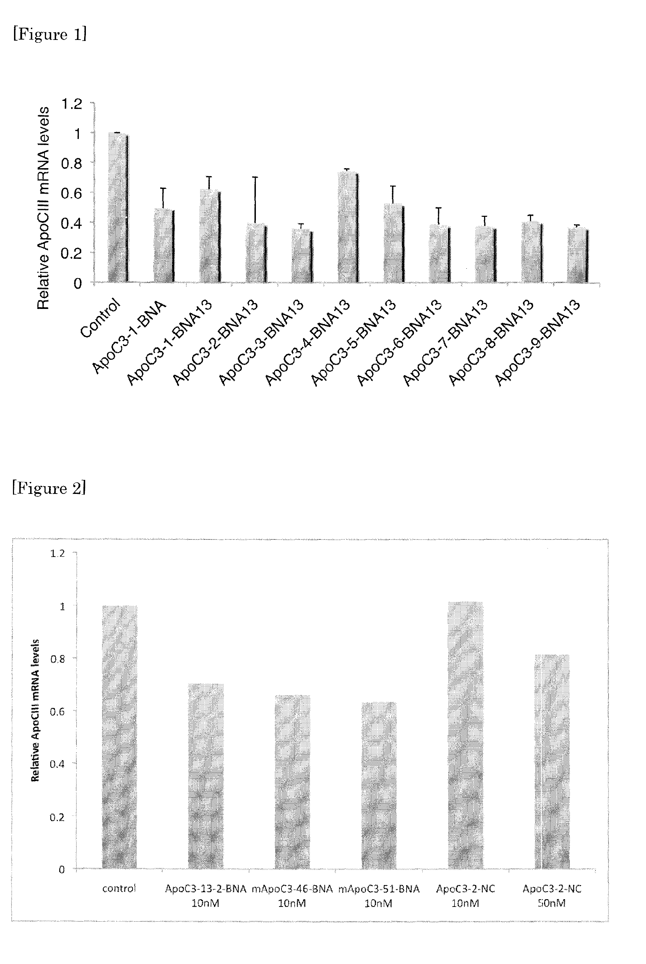

The cDNA was prepared from 10 µg of the total RNA using High Capacity cDNA Reverse Transcription Kit (manufactured by Applied Biosystems). Using the obtained cDNA and Taqman Universal PCR Master Mix (manufactured by Applied Biosystems), real-time PCR was performed to quantify the ApoC3 mRNA level. In the real-time PCR, the mRNA level of GAPDH, which is the housekeeping gene, was also quantified at the same time, and the ApoC3 mRNA level with respect to the GAPDH mRNA level was evaluated. FIG. 1 below shows the results in which the control mRNA level was taken as 1.

ApoC3-1-S, ApoC3-1-BNA, ApoC3-1-BNA13, ApoC3-2-BNA13, ApoC3-3-BNA13, ApoC3-4-BNA13, ApoC3-5-BNA13, ApoC3-6-BNA13, ApoC3-7-BNA13, ApoC3-8-BNA13 and ApoC3-9-BNA13 were used as the oligonucleotide. Mm00445670_m1 (manufactured by Applied Biosystems) was used as a Taqman probe for quantifying the ApoC3 mRNA level, and Mm99999915_g1 (manufactured by Applied Biosystems) was used as a Taqman probe for quantifying the GAPDH mRNA level.

As is clear from FIG. 1, the ApoC3 mRNA expression level in the mouse NMuli cells treated with the oligonucleotides was lower than that in the untreated mouse NMuli cells. That is, it was found that all oligonucleotides used suppress the ApoC3 gene expression.

### Oligonucleotide addition experiment on human hepatocyte

The experiment was performed in the same manner as described above, except that human liver cell strain Huh-7 cells were used in place of mouse liver cell strain NMuli cells and the experiment was performed also at a final concentration of the oligonucleotide of 10 nM in addition to 50 nM. FIG. 2 shows the results. It should be noted that ApoC3-2-BNA13, ApoC3-46-BNA14, ApoC3-51-BNA14 and ApoC3-2-NC were used as the oligonucleotide.

As is clear from FIG. 2, the ApoC3 mRNA expression level in the human Huh-7 cells treated with the oligonucleotides was lower than that in the untreated human Huh-7 cells. That is, it was found that all oligonucleotides used suppress the ApoC3 gene expression.

### Example 2: Suppression of ApoC3 gene expression in vivo by oligonucleotide - (1)

### Oligonucleotide administration experiment on mouse

Five 6-week old C57BL6/J mice (male: CLEA Japan) were provided as test animals for each administration group. After the mice were fed with a high-fat diet (F2WTD1: containing 0.3% cholesterol, manufactured by Oriental Yeast Co., Ltd.) for 2 weeks, the oligonucleotide ApoC3-1-BNA or ApoC3-1-S, or saline (control) was intraperitoneally administered (20 mg/kg/dose). Administration was performed twice per week, five doses in total. The blood was collected from the tail vein in a fasting state two weeks after the last administration. Next, the mice were anesthetized with diethyl ether and then were subjected to perfusion with PBS from the heart. The liver was taken, was washed with PBS, was cut into small pieces, was instantaneously frozen with liquid nitrogen, and then stored at -80°C.

### Quantification of serum triglyceride level

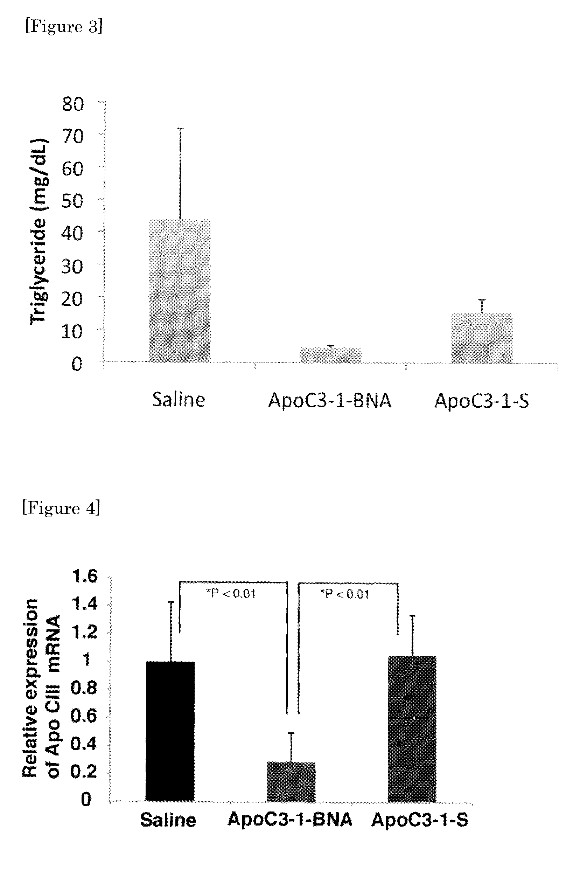

The blood collected from the mouse tail vein was allowed to stand for 20 minutes at room temperature, and then was centrifuged at 5,000 rpm at 4°C for 20 minutes to separate the serum. The serum triglyceride level of each serum sample was quantified using Triglyceride E-Test Wako (manufactured by Wako Pure Chemical Industries, Ltd.). Three hundred microliters of a color-producing reagent was added to 2 µL of the serum, the mixture was warmed at 37°C for 5 minutes, and the absorbance was measured at 600 nm using a spectrophotometer. A value was calculated using the calibration curve of a standard reagent. FIG. 3 shows the results.

As is clear from FIG. 3, the serum triglyceride level (TG) in the oligonucleotide ApoC3-1-BNA administered group was approximately 15% of that in the control group. TG in the oligonucleotide ApoC3-1-S administered group was approximately 46% of that in the control group. It was found that the oligonucleotide including ApoC3-1-BNA is effective in reducing TG and the like.

### Extraction and quantification of mRNA from liver: Real-time PCR

The frozen liver sections were homogenized in 1 mL of TRIzol Regent (manufactured by Invitrogen), 200 µL of chloroform was added thereto, and then, the mixture was centrifuged at 13,200 rpm at 4°C for 15 minutes. Two hundred and twenty microliters of supernatant was added to 400 µL of isopropanol. The mixture was mixed by inversion and was centrifuged at 13,200 rpm at 4°C for 15 minutes, and then, isopropanol was removed. Next, 800 µL of 75% ethanol was added, and then, the mixture was centrifuged at 13,200 rpm at 4°C for 5 minutes. The precipitate containing total RNA was dissolved in 80 µL of RNA-free water (Water, DEPC treated, RNase tested; Nacalai Tesque, Inc.). The extracted total RNA was quantified with a spectrophotometer, and the length of the RNA was confirmed by agarose gel electrophoresis.

The cDNA was prepared from 10 µg of the total RNA using High Capacity cDNA Reverse Transcription Kit (manufactured by Applied Biosystems). Using the obtained cDNA and Taqman Universal PCR Master Mix (manufactured by Applied Biosystems), real-time PCR was performed to quantify the mouse ApoC3 mRNA level. In the real-time PCR, the mRNA level of GAPDH, which is the housekeeping gene, was also quantified at the same time, and the mouse ApoC3 mRNA level with respect to the GAPDH mRNA level was evaluated. FIG. 4 below shows the results in which the control mRNA level was taken as 1.

Mm00445670_m1 (manufactured by Applied Biosystems) was used as a Taqman probe for quantifying the ApoC3 mRNA level, and Mm99999915_g1 (manufactured by Applied Biosystems) was used as a Taqman probe for quantifying the GAPDH mRNA level.

As is clear from FIG. 4, the ApoC3 mRNA level in the oligonucleotide ApoC3-1-BNA administered group is significantly lower than that in the control group. The mRNA level in the oligonucleotide ApoC3-1-S administered group is the same as that in the control group.

### Quantification of ApoC3 protein: Western blotting

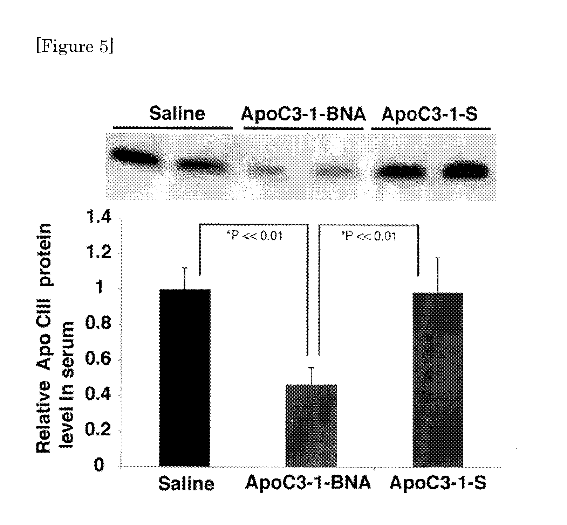

The serum protein level was quantified using Bio-Rad DC (manufactured by Bio-Rad Laboratories, Inc.). The serum (50 µg of protein) was applied to the respective lanes on Novex (registered trademark) Tris-Glycine Gels (16%) (manufactured by Invitrogen), and electrophoresis was performed at 200 V for 40 minutes. Blotting was performed at 180 mA for 90 minutes using Immun-Blot (registered trademark) PVDF Membrane (manufactured by Bio-Rad Laboratories, Inc.), and then blocking was performed for 1 hour using Blocking One (Nacalai Tesque, Inc.). The obtained membrane was reacted with a rabbit anti-apoC-III polyclonal antibody (apoC-III M-75, Santa Cruz Biotechnology Inc.) as a primary antibody, and reacted with an anti-rabbit polyclonal antibody (Goat anti rabbit IgG HRP, Santa Cruz Biotechnology Inc.) as a secondary antibody. Then, the membrane was allowed to develop a color using ECL plus (Western Blotting Detection System, GE Healthcare), and the mouse ApoC3 protein level was quantified. FIG. 5 shows the results.

As is clear from FIG. 5, the ApoC3 protein level in the oligonucleotide ApoC3-1-BNA administered group was remarkably lower than that in the control group.

As described above, it was found that the oligonucleotide including ApoC3-1-BNA is effective in reducing TG and the like by suppressing the ApoC3 gene expression.

### Example 3: Suppression of ApoC3 gene expression in vivo by oligonucleotide - (2)

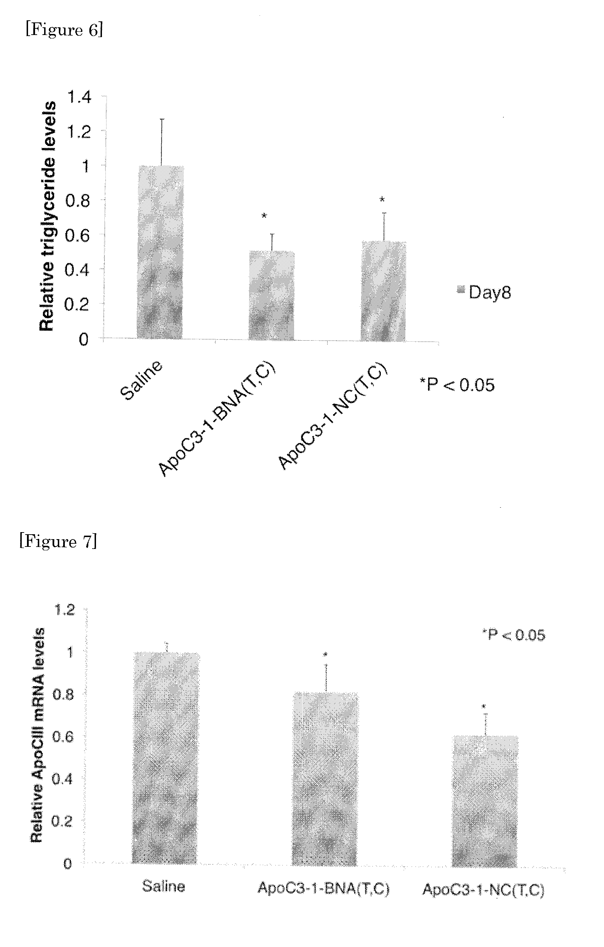

The experiment was performed in the same manner as in Example 2, except that ApoC3-1-BNA (T, C) or ApoC3-1-NC (T, C) was used as the oligonucleotide, the dosage was 10 mg/kg/dose instead of 20 mg/kg/dose, and the blood and the liver were taken one week after the last administration instead of two weeks after the last administration. FIGS. 6 to 8 show the results.

As is clear from FIG. 6, the serum triglyceride levels (TG) in the oligonucleotide ApoC3-1-BNA (T, C) administered group and the ApoC3-1-NC (T, C) administered group were approximately 50% of that in the control group. It was found that the oligonucleotides including ApoC3-1-BNA and ApoC3-1-NC are effective in reducing TG.

As is clear from FIG. 7, the ApoC3 mRNA level in the oligonucleotide ApoC3-1-BNA (T, C) administered group was approximately 80% of that in the control group, and the ApoC3 mRNA level in the ApoC3-1-NC (T, C) administered group was approximately 60% of that in the control group.

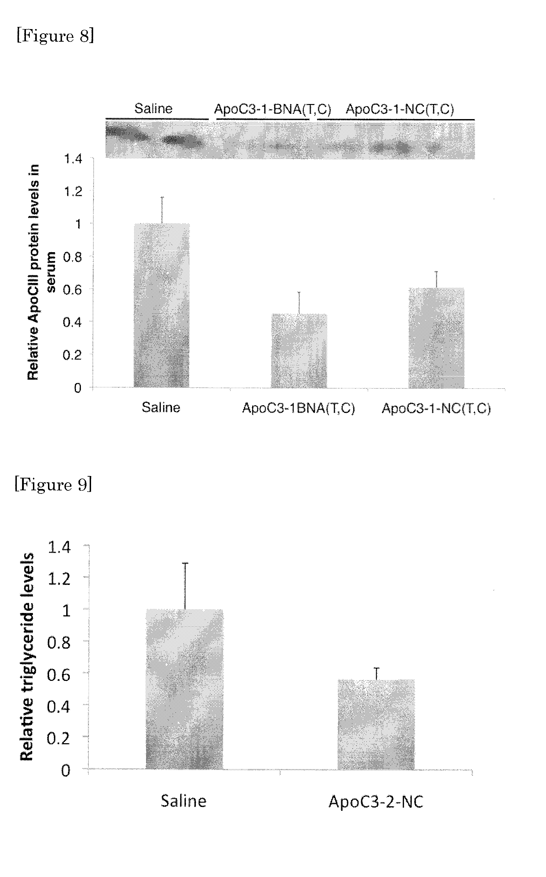

As is clear from FIG. 8, the ApoC3 protein levels in the oligonucleotide ApoC3-1-BNA (T, C) administered group and the ApoC3-1-NC (T, C) administered group were remarkably lower than that in the control group.

As described above, it was found that the oligonucleotides including ApoC3-1-BNA and ApoC3-1-NC are effective in reducing TG by suppressing the ApoC3 gene expression. No hepatotoxicity was observed by measuring the level of AST and ALT in serum using Transaminase CII-Test Wako (manufactured by Wako Pure Chemical Industries, Ltd.).

### Example 4: Suppression of ApoC3 gene expression in vivo by oligonucleotide - (3)

The experiment was performed in the same manner as in Example 2, except that ApoC3-2-NC was used as the oligonucleotide and the dosage was 5 mg/kg/dose instead of 20 mg/kg/dose. FIGS. 9 to 11 show the results.

As is clear from FIG. 9, the serum triglyceride level (TG) in the oligonucleotide ApoC3-2-NC administered group was approximately 60% of that in the control group. It was found that the oligonucleotide including ApoC3-2-NC is effective in reducing TG.

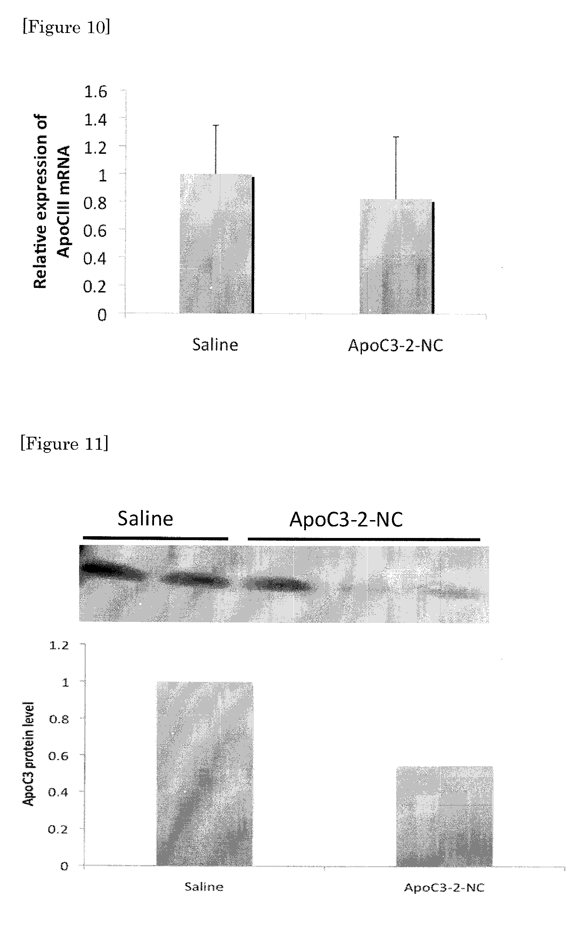

As is clear from FIG. 10, the ApoC3 mRNA level in the oligonucleotide ApoC3-2-NC administered group was approximately 80% of that in the control group.

As is clear from FIG. 11, the ApoC3 protein levels in the oligonucleotide ApoC3-2-NC administered group and the ApoC3-1-NC (T, C) administered group were approximately 60% of that in the control group.

As described above, it was found that the oligonucleotide including ApoC3-2-NC is effective in reducing TG by suppressing the ApoC3 gene expression. No hepatotoxicity was observed from the level of AST and ALT in serum.

### Example 5: Suppression of ApoC3 gene expression in vivo by oligonucleotide - (4)

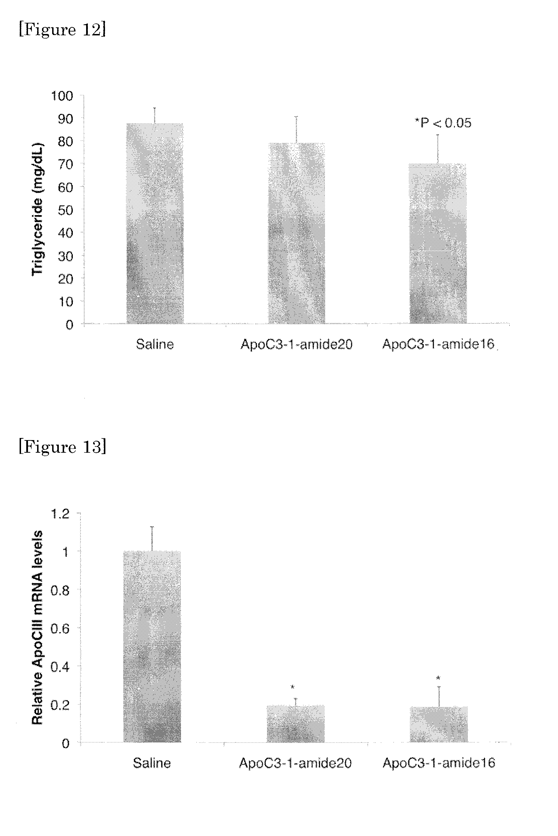

The experiment was performed in the same manner as in Example 2, except that ApoC3-1-amide20 or ApoC3-1-amide16 was used as the oligonucleotide and the oligonucleotide was administered into the tail vein twice per three days (20 mg/kg/dose) instead of being intraperitoneally administered twice per week, five times in total (20 mg/kg/dose). FIGS. 12 to 14 show the results.

As is clear from FIG. 12, TG in the oligonucleotide ApoC3-1-amide20 administered group was approximately 90% of that in the control group and TG in the oligonucleotide ApoC3-1-amide16 administered group was approximately 80% of that in the control group. It was found that the oligonucleotide including ApoC3-1-AM is effective in reducing TG.

As is clear from FIG. 13, the ApoC3 mRNA levels in the oligonucleotide ApoC3-1-amide20 administered group and the oligonucleotide ApoC3-1-amide16 administered group were approximately 20% of that in the control group.

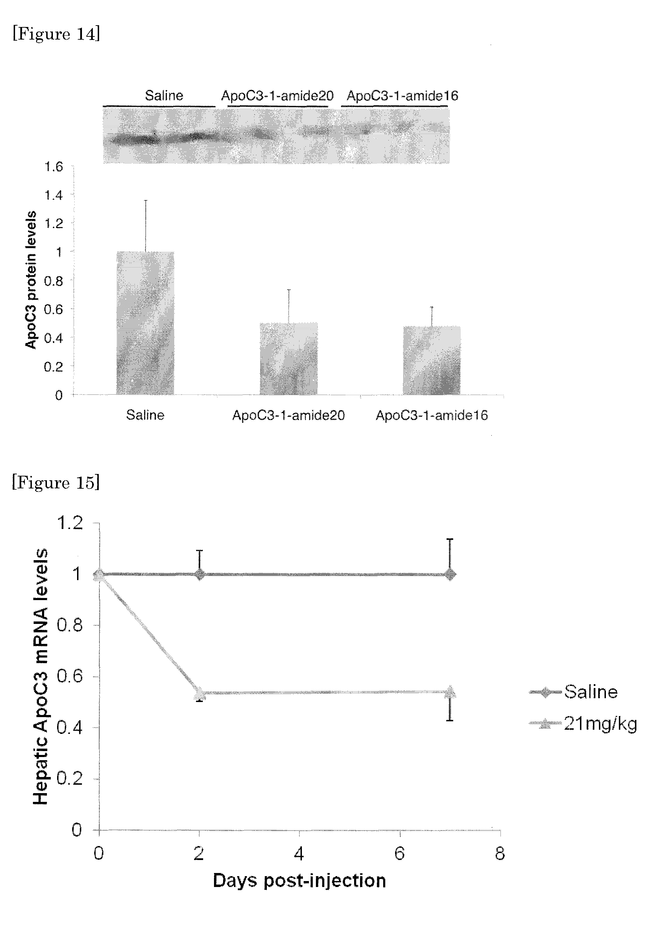

As is clear from FIG. 14, the ApoC3 protein levels in the oligonucleotide ApoC3-1-amide20 administered group and the oligonucleotide ApoC3-1-amide16 administered group were approximately 50% of that in the control group.

As described above, it was found that the oligonucleotide including ApoC3-2-AM is effective in reducing TG by suppressing the ApoC3 gene expression.

### Example 6: Suppression of ApoC3 gene expression and reduction of serum TG level by single intravenous administration of oligonucleotide

An oligonucleotide ApoC3-13-2-BNA (CTGcatggcaCCT: the 5' end of the sequence is represented in the left side. The small letter represents a native DNA, and the capital letter represents 2',4'-BNA/LNA. All of the phosphate backbones were phosphorothioated. The base sequence is shown as Sequence ID No. 17.) complementary to the target region from the positions 46 to 58 of the mouse ApoC3 gene (Sequence ID No. 2) or the positions 40 to 52 of the human ApoC3 gene (Sequence ID No. 1) was prepared according to the section "Preparation of oligonucleotide" described in Example 1.

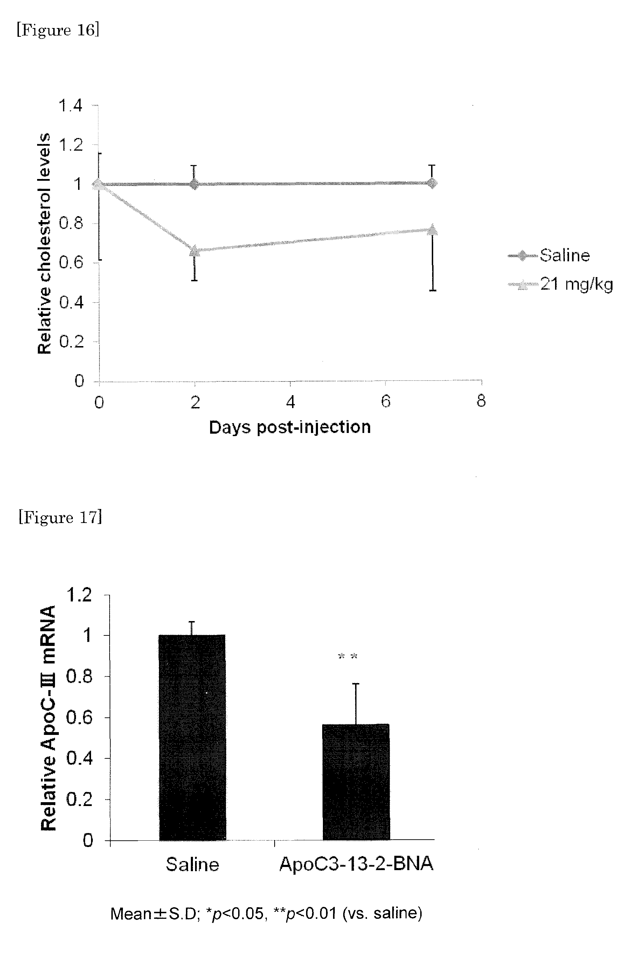

The experiment was performed in the same manner as in Example 2, except that 8-week old C57BL6/J mice as test animals were fed with a high-fat diet (F2WTD1) for 3 weeks, ApoC3-13-2-BNA was used as the oligonucleotide, the oligonucleotide was administered once into the tail vein (21 mg/kg/dose) and the blood and the liver were taken two and seven days after the administration. FIGS. 15 and 16 show changes over time in an ApoC3 mRNA level in the liver after the administration and changes over time in a serum triglyceride level, respectively.

As is clear from FIG. 15, the ApoC3 mRNA level in the oligonucleotide ApoC3-13-2-BNA administered group was approximately 50% of that of the control group two days after the administration, and therefore, the gene suppression effect was confirmed.

As is clear from FIG. 16, the TG in the oligonucleotide ApoC3-13-2-BNA administered group was reduced by approximately 40% two days after the administration, and the effect was exhibited even a week after the administration.

As described above, it was observed that the effect lasts for a week or more by a single administration of the oligonucleotide.

### Example 7: Suppression of ApoC3 gene expression, reduction of serum TG level and improvement of metabolism in chronic hypertriglyceridemic ApoE knockout mouse by administration of oligonucleotide

Three 6-week old ApoE knockout mice (male: model animals of chronic hypertriglyceridemia obtained by in-house breeding.) were provided as test animals for each administration group, and were fed with a high-fat diet (F2WTD1) for 2 weeks. ApoC3-13-2-BNA was used as the oligonucleotide. The blood was collected in a fasting state two days before administration of the oligonucleotide. ApoC3-13-2-BNA or saline was subcutaneously administered in six doses, that is, once per week, five doses in total, and then once per two weeks (10mg/kg/dose). During that period, the blood was collected several times from the tail vein. Then, the liver was taken on the last day. The ApoC3 mRNA level in the liver was quantified and the total TG level in the serum was measured using the procedures in Example 2. The concentration of adiponectin in mouse serum was quantified using CircuLex™ Mouse Adiponectin ELISA Kit (manufactured by Medical & Biological Laboratories Co., Ltd.) according to the protocol.

FIGS. 17 to 19 show the ApoC3 mRNA level in the liver after the administration was completed, changes over time in the serum triglyceride level after the administration was started, and the concentration of adiponectin in serum after the administration was completed, respectively.

As is clear from FIG. 17, the ApoC3 mRNA level in the oligonucleotide ApoC3-13-2-BNA administered group was reduced to approximately 50% of that in the control group three months after the administration.

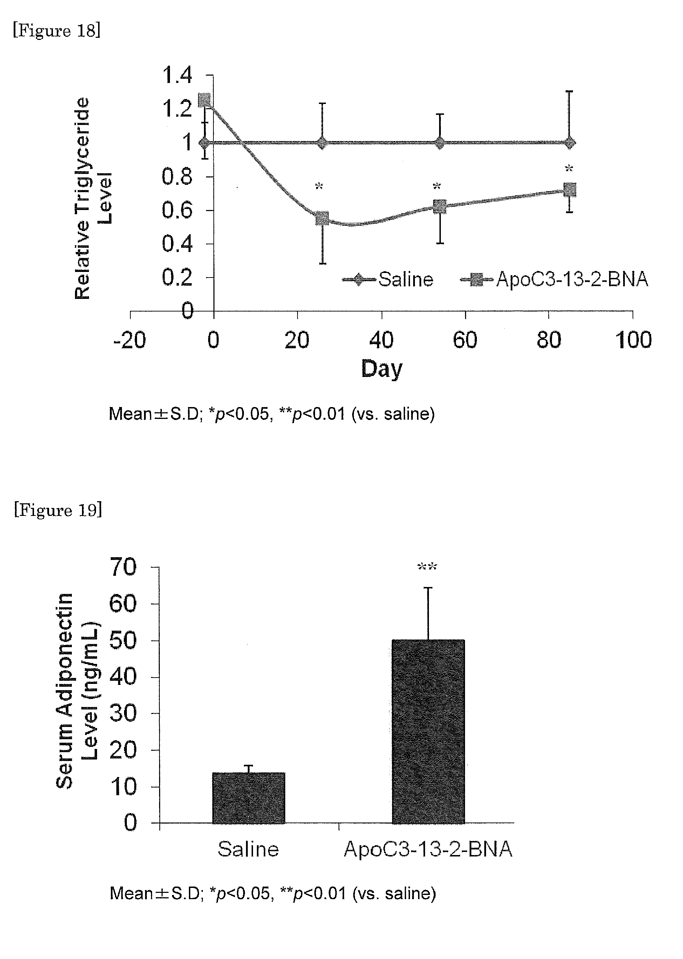

As is clear from FIG. 18, the total TG level in serum in the oligonucleotide ApoC3-13-2-BNA administered group was remarkably reduced a week after the administration, and was reduced on average by 40% throughout after that.

As is clear from FIG. 19, in the oligonucleotide ApoC3-13-2-BNA administered group, adiponectin protein in blood which is a suppressor of arteriosclerosis produced from fat was increased approximately three times compared with that in the control group, and the improvement of metabolism was confirmed.

Moreover, as a result of histopathological analysis, although a symptom of a fatty liver was observed in the liver in both groups, it was confirmed that a state of a fatty liver is significantly milder in the oligonucleotide ApoC3-13-2-BNA administered group.

### Example 8: Study using sugar moiety bridged artificial nucleic acid 2',4'-BNA^{AM} [N-R]

In this example, analogues of a sugar moiety bridged artificial nucleic acid 2',4'-BNA^{AM} [N-R] in which R was any substituent such as a methyl group (Me), isopropyl group (iPR), benzyl group (Bn) or the like were synthesized and their medicinal efficacy in vivo was studied.

Various oligonucleotides shown in Table 2 below were designed and prepared. The oligonucleotides were prepared according to the section "Preparation of oligonucleotide" described in Example 1. All of the oligonucleotides have the base sequence of Sequence ID No. 5.

**Table 2**

| Oligonucleotide name | Sequence of Oligonucleotide | Target Region of Mouse ApoC3 Gene | Sequence ID No. |
|---|---|---|---|
| ApoC3-1-BNA-16 | TTaTccagcttTaTTa | 497-512 | 5 |
| ApoC3-1-Am_Me-16 | TₐTₐaTₐccagcttTₐaTₐTₐa | | |
| ApoC3-1-Am_Bn-16 | T_{b}T_{b}aT_{b}ccagcttT_{b}aT_{b}T_{b}a | | |
| ApoC3-1-Am_Me_Bn-16 | T_{b}TₐaTₐccagcttTₐaTₐT_{b}a | | |
| ApoC3-1-Am_iPr-16 | T_{c}T_{c}aT_{c}ccagcttT_{c}aT_{c}T_{c}a | | |

| | | | |
|---|---|---|---|
| *5'end of the sequence is represented in the left side. The small letter represents a native DNA, the capital letter represents 2',4'-BNA/LNA (BNA) , the capital letters with subscript a to c represent 2',4'-BNA^{AM}[N-R](AM), wherein in the subscript a, R=Me, in the subscript b, R =Bn, in the subscript c, R=iPr. All of the phosphate backbones are phosphorothioated. | | | |

Four 9-week old C57BL6/J mice were provided as test animals for each administration group, and while fed with a normal diet (manufactured by CLEA Japan, Inc.), administered with each oligonucleotide in Table 2 once into their tail vein (2.84 µmol/kg/dose), and then the blood and the liver were taken three days after the administration. The ApoC3 mRNA level in the liver was quantified using the procedure in Example 2. The oligonucleotide molecules accumulated in the liver were quantified by an ELISA applied approach described below. The levels of transaminases (AST and ALT) in the obtained serum were quantified as described below.

The oligonucleotide molecules in the liver were quantified as follows: the liver tissue sections of the administered mouse were homogenized in 1 mL of RIPA buffer (manufactured by Aldrich) and were subjected to refrigerated centrifugation at 10,000 rpm for 3 minutes, and the supernatant was subjected to protein quantification using Bio-Rad DC protein assay (manufactured by Bio-Rad Laboratories, Inc.). Ten microliters of each supernatant, a probe (5'-gaa tag cga taa taa agc tgg ata a-3': 3' end is biotinated; Sequence ID No. 18) and a template DNA (5'-tcg cta ttc-3': 5' end is phosphorylated and 3' end modified with digoxigenin; Sequence ID No. 19) were dispensed to a streptavidin coated 96-well plate (manufactured by Nunc) and were incubated at 37°C for 1 hour. The solution in each well was removed, the well was washed, and a digoxigenin-labeled, phosphorylated DNA probe and T4 DNA ligase (manufactured by Takara Bio Inc.) were put into the well and were incubated at 15°C for 2 hours. The solution in each well was removed, the well was washed, and anti-digoxigenin antibody (manufactured by Roche) was added and incubated at 37°C for 1 hour. The solution in each well was removed, the well was washed, CDP-star (Applied Biosystems) was added and light emission was quantified.

The AST level and ALT level were quantified using Transaminase CII-Test Wako (manufactured by Wako Pure Chemical Industries, Ltd.). Two hundred and fifty microliters of a substrate enzyme liquid for AST level measurement or ALT level measurement was added to 10 µL of the serum, and the mixture was warmed at 37°C for 5 minutes. Two hundred and fifty microliters of a color-producing reagent was added, and the mixture was warmed at 37°C for 20 minutes. Next, after adding 1 mL of a reaction stop solution, the absorbance was measured at 555 nm using a spectrophotometer. The respective values were calculated using the calibration curve of a standard reagent.

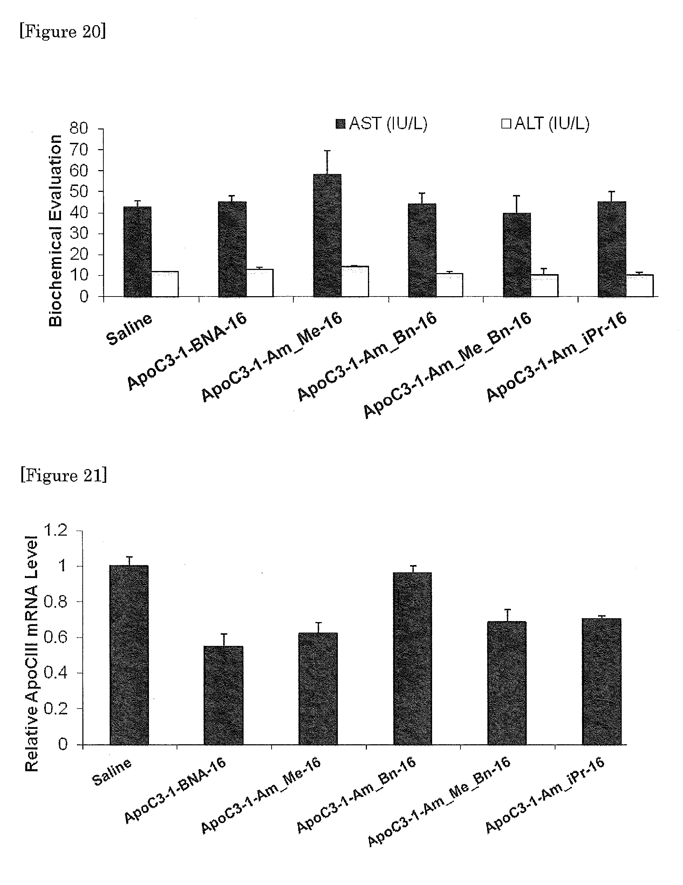

FIGS. 20 to 22 show the serum transaminase levels (the AST level on the left and the ALT level on the right in each administered group), the ApoC3 mRNA levels in the liver and the oligonucleotide nucleic acid levels in the liver, respectively after the administration was completed.

As is clear from FIG. 20, it was observed that there are no large differences in the serum transaminase levels between the administered groups.

As is clear from FIG. 21, the ApoC3 gene expression suppressing effect was the largest in the mRNA oligonucleotide ApoC3-1-BNA-16 administered group, in which the expression was suppressed by approximately 50%. The ApoC3-1-Am_Me-16 administered group exhibited an expression suppressing effect equivalent to that in the ApoC3-1-BNA-16 administered group. In turn, the expression suppressing effect was exhibited in the order of ApoC3-1-Am_Me_Bn-16 and ApoC3-1-Am_iPr-16. ApoC3-1-Am_Bn-16 apparently exhibited no suppression effect.

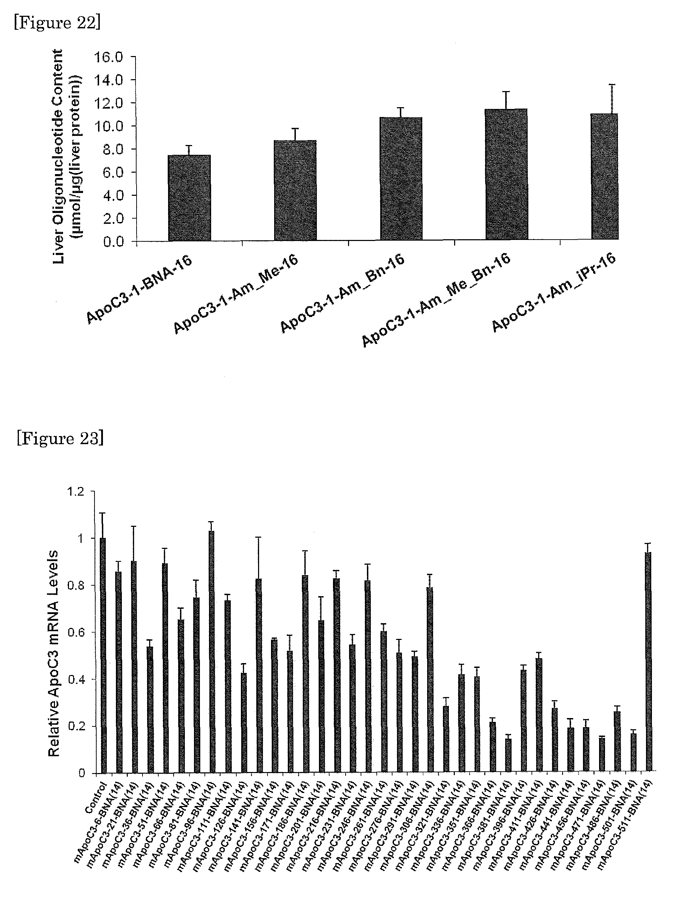

As is clear from FIG. 22, it was observed that the nucleic acid accumulation level of each oligonucleotide in the target liver was high in the groups administered with ApoC3-1-Am_iPr-16, ApoC3-1-Am_Bn-16 and ApoC3-1-Am_Me_Bn-16, which contain an isopropyl group (iPr) or benzyl group (Bn) that is a substituent with high hydrophobicity. It is assumed that a mismatch between the accumulation level and the medicinal efficacy is caused by the reduction of recognition ability of RNase H, which is an enzyme required in the antisense method, due to bulkiness of the substituent.

As described above, it was suggested that 2',4'-BNA^{AM} [N-R] exhibits the same effect as 2',4'-BNA/LNA, and can provide better medicinal efficacy and pharmacokinetics than 2',4'-BNA/LNA by appropriately determining a substituent of 2',4'-BNA^{AM} [N-R].

### Example 9: Oligonucleotide addition experiment on mouse primary hepatocyte

In this example, antisense molecules including 2',4'-BNA/LNA to be uniformly distributed on the entire ApoC3 mRNA were evaluated in vitro.

Various oligonucleotides shown in Tables 3 and 4 below were prepared (the corresponding base sequences of the oligonucleotides shown as Sequence ID Nos. 16 and 20 to 53 as shown in these tables). These oligonucleotides were designed by limiting the sequence length to 14-mer and selecting target regions regularly from the base sequence of the mouse ApoC3 gene (Sequence ID No. 2) using mApoC3-{15n-9}-BNA(14) (n=1-35). In Tables 3 and 4, the target regions of the mouse ApoC3 gene (Sequence ID No. 2) are also shown. The oligonucleotide mApoC3-51-BNA(14) also targets the region from the positions 44 to 57 of the human ApoC3 gene (Sequence ID No. 1). The preparation of oligonucleotides was in the same manner as in Example 1.

**Table 3**

| Oligonucleotide name | Sequence of Oligonucleotide | Target Region of Mouse ApoC3 Gene | Sequence ID No. |
|---|---|---|---|
| mApoC3-6-BNA(14) | TAGggataaaaCTg | 6-19 | 20 |
| mApoC3-21-BNA(14) | GTAgctagctgCTt | 21-34 | 21 |
| mApoC3-36-BNA(14) | ACCtacgtaccTGg | 36-49 | 22 |
| mApoC3-51-BNA(14) | CCGgggctgcaTGg | 51-64 | 16 |
| mApoC3-66-BNA(14) | CACagtgaggaGCg | 66-79 | 23 |
| mApoC3-81-BNA(14) | GAGagccaagaGGg | 81-94 | 24 |
| mApoC3-96-BNA(14) | TCGggcagatgCCa | 96-109 | 25 |
| mApoC3-111-BNA(14) | CTCtacctcttCAg | 111-124 | 26 |
| mApoC3-126-BNA(14) | CAGcagcaaggATc | 126-139 | 27 |
| mApoC3-141-BNA(14) | GCCctgtacagAGc | 141-154 | 28 |
| mApoC3-156-BNA(14) | GGCttgttccaTGt | 156-169 | 29 |
| mApoC3-171-BNA(14) | CTGgaccgtctTGg | 171-184 | 30 |
| mApoC3-186-BNA(14) | GCTacttagcgCAt | 186-199 | 31 |
| mApoC3-201-BNA(14) | ATCggactcctGCa | 201-214 | 32 |
| mApoC3-216-BNA(14) | GGCcaccacagCTa | 216-229 | 33 |
| mApoC3-231-BNA(14) | GTCcatccagcCCc | 231-244 | 34 |
| mApoC3-246-BNA(14) | GAAtctgaagtGAt | 246-259 | 35 |
| mApoC3-261-BNA(14) | CCAgtagccttTCa | 261-274 | 36 |
| mApoC3-276-BNA(14) | GTCagtaaactTGc | 276-289 | 37 |
| mApoC3-291-BNA(14) | GAAgccggtgaACt | 291-304 | 38 |

| | | | |
|---|---|---|---|
| *5'end of the sequence is represented in the left side. The small letter represents an native DNA, and the capital letter represents 2',4'-BNA/LNA(BNA). All of the phosphate backbones are phosphorothioated. | | | |

**Table 4**

| Oligonucleotide name | Sequence of Oligonucleotide | Target Region of Mouse ApoC3 Gene | Sequence ID No. |
|---|---|---|---|
| mApoC3-306-BNA(14) | AGGgttagaatCCc | 306-319 | 39 |
| mApoC3-321-BNA(14) | AGTtggttggtCCt | 321-334 | 40 |
| mApoC3-336-BNA(14) | CGActcaatagCTg | 336-349 | 41 |
| mApoC3-351-BNA(14) | AACacagaagtCTc | 351-364 | 42 |
| mApoC3-366-BNA(14) | AACaggcacatCTg | 366-379 | 43 |
| mApoC3-381-BNA(14) | GCAgcaggatgGAg | 381-394 | 44 |
| mApoC3-396-BNA(14) | CAGgcctggagGGg | 396-409 | 45 |
| mApoC3-411-BNA(14) | TCAggggccacCTg | 411-424 | 46 |
| mApoC3-426-BNA(14) | CCCttaaagcaACc | 426-439 | 47 |
| mApoC3-441-BNA(14) | TGAgaacatacTTt | 441-454 | 48 |
| mApoC3-456-BNA(14) | GGAggggtgaaGAc | 456-469 | 49 |
| mApoC3-471-BNA(14) | TTAggtgagatCTa | 471-484 | 50 |
| mApoC3-486-BNA(14) | TTAgggacagcATg | 486-499 | 51 |
| mApoC3-501-BNA(14) | TCTtatccagcTTt | 501-509 | 52 |
| mApoC3-511-BNA(14) | AACagcagcttCTt | 511-524 | 53 |

| | | | |
|---|---|---|---|
| *5'end of the sequence is represented in the left side. The small letter represents an native DNA, and the capital letter represents 2',4'-BNA/LNA(BNA). All of the phosphate backbones are phosphorothioated. | | | |

Mouse primary hepatocytes prepared so as to have 3.3×10⁵ cells/mL in Williams' Medium E (manufactured by Sigma; with 5% FBS, 1% penicillin/streptomycin and 1% Glutamax) were seeded onto a 96-well plate in an amount of 100 µL per well, and cultured at 37°C under 5% CO₂ for 24 hours. In order to set a final concentration of the oligonucleotide to 10 nM, 11 µL of a 100 nM oligonucleotide-containing solution was mixed with 0.55 µL of Lipofectamine 2000 (manufactured by Invitrogen) and 38.45 µL of Opti-MEM (manufactured by Invitrogen), then the mixed solution was incubated at room temperature for 20 minutes, and 50 µL thereof and 50 µL of Opti-MEM were added to each well. The culture medium was replaced 4 hours after the addition of oligonucleotide. Twenty more hours later, the cDNA was prepared from total RNA in the cell lysate using Cells-to-CT Kit (manufactured by Applied Biosystems). Cells cultured in the medium to which no oligonucleotide was added were used as a control. The mApoC3 mRNA level was quantified using the obtained cDNA in the same manner as in Example 1. FIG. 23 shows the results.

As is clear from FIG. 23, in regard to almost all the oligonucleotides except the mApoC3-96-BNA(14) (oligonucleotide of which the corresponding base sequence is shown as Sequence ID No. 25), the ApoC3 mRNA expression level in the mouse hepatocytes treated with the oligonucleotide was lower than that in the untreated control cells without the addition of the oligonucleotide, and the expression suppressing effect was exhibited. Furthermore, in the mouse hepatocytes treated with the oligonucleotide of which the corresponding base sequence is shown as Sequence ID No. 22, 27, 30, 34, 38, or 40 to 52, the ApoC3 mRNA expression level was extremely low, and the particularly high expression suppressing effect was exhibited. The expression suppressing effect was well observed, for example, in the case where the target region of the oligonucleotide is located in 3'-untranslated region (3' UTR) (for example, the oligonucleotides of which the corresponding base sequences are shown as Sequence ID Nos. 40 to 52).

### Industrial Applicability

According to the present study, an oligonucleotide useful as a therapeutic agent for hyperlipidemia can be provided that is excellent in binding affinity to the apolipoprotein C-III gene, stability and safety. The oligonucleotide of the present invention is promising in the prevention of coronary disease such as myocardial infarction or cerebrovascular disease.

## Claims

1. An oligonucleotide comprising a sugar-modified nucleoside,
the sugar-modified nucleoside having a cross-linked structure between 4'-position and 2'-position, and
the oligonucleotide being capable of binding to an apolipoprotein C-III gene.

2. The oligonucleotide according to claim 1, wherein the cross-linked structure is represented by -CO-NR¹-, -CH₂-CO-NR¹-, -(CH₂)₂-CO-NR¹-, -CO-NR¹-X-, or -CH₂-CO-NR¹-X-,
where R¹ represents a hydrogen atom;
an alkyl group having 1 to 7 carbon atoms that may be branched or form a cyclic group;
an alkenyl group having 2 to 7 carbon atoms that may be branched or form a cyclic group;
an aryl group having 3 to 12 carbon atoms that may have any one or more substituents selected from the group α consisting of a hydroxyl group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms and a halogen atom, and may contain a heteroatom; or
an aralkyl group with an aryl moiety having 3 to 12 carbon atoms that may have any one or more substituents selected from the group α and may contain a heteroatom; and
X represents an oxygen atom, a sulfur atom, an amino group or a methylene group.

3. The oligonucleotide according to claim 1, wherein the cross-linked structure is represented by -CH₂-O-, -(CH₂)₂-O-, -CH₂-NR¹-O-, or -(CH₂)₂-NR¹-O-,
where R¹ represents a hydrogen atom;
an alkyl group having 1 to 7 carbon atoms that may be branched or form a cyclic group;
an alkenyl group having 2 to 7 carbon atoms that may be branched or form a cyclic group;
an aryl group having 3 to 12 carbon atoms that may have any one or more substituents selected from the group α consisting of a hydroxyl group, linear alkyl groups having 1 to 6 carbon atoms, linear alkoxy groups having 1 to 6 carbon atoms, a mercapto group, linear alkylthio groups having 1 to 6 carbon atoms, an amino group, linear alkylamino groups having 1 to 6 carbon atoms and a halogen atom, and may contain a heteroatom; or
an aralkyl group with an aryl moiety having 3 to 12 carbon atoms that may have any one or more substituents selected from the group α and may contain a heteroatom.

4. The oligonucleotide according to any one of claims 1 to 3, wherein the apolipoprotein C-III gene is a human apolipoprotein C-III gene.

5. The oligonucleotide according to any one of claims 1 to 4, wherein the oligonucleotide is capable of binding to a 3'-untranslated region of the apolipoprotein C-III gene.

6. The oligonucleotide according to any one of claims 1 to 4, wherein a base sequence that becomes a basis for the oligonucleotide includes any one of the base sequences of Sequence ID Nos. 3 to 17, 20 to 24, and 26 to 53.

7. The oligonucleotide according to any one of claims 1 to 6, wherein the oligonucleotide has a base sequence length of 10 to 25 bases.

8. A therapeutic agent for hyperlipidemia, comprising an oligonucleotide of any one of claims 1 to 7 as an active ingredient.


SEQUENCE LISTING

<110>  Osaka University
       National Cerebral and Cardiovascular Center

<120>  Oligonucleotide and therapeutic agent for hyperlipidemia  containing
the same as an active ingredient

<130>  P2-12O07141

<150>  JP 2011-271751
<151>  2011-12-12

<160>  53

<170>  PatentIn version 3.1

<210>  1
<211>  533
<212>  DNA
<213>  Homo sapiens

<400>  1
tgctcagttc atccctagag gcagctgctc caggaacaga ggtgccatgc agccccgggt      60

actccttgtt gttgccctcc tggcgctcct ggcctctgcc cgagcttcag aggccgagga     120

tgcctccctt ctcagcttca tgcagggtta catgaagcac gccaccaaga ccgccaagga     180

tgcactgagc agcgtgcagg agtcccaggt ggcccagcag gccaggggct gggtgaccga     240

tggcttcagt tccctgaaag actactggag caccgttaag gacaagttct ctgagttctg     300

ggatttggac cctgaggtca gaccaacttc agccgtggct gcctgagacc tcaatacccc     360

aagtccacct gcctatccat cctgcgagct ccttgggtcc tgcaatctcc agggctgccc     420

ctgtaggttg cttaaaaggg acagtattct cagtgctctc ctaccccacc tcatgcctgg     480

cccccctcca ggcatgctgg cctcccaata aagctggaca agaagctgct atg             533


<210>  2
<211>  524
<212>  DNA
<213>  Mus musculus

<400>  2
ctgctcagtt ttatccctag aagcagctag ctactccagg tacgtaggtg ccatgcagcc      60

ccggacgctc ctcactgtgg ccctcttggc tctcctggca tctgcccgag ctgaagaggt     120

agagggatcc ttgctgctgg gctctgtaca gggctacatg gaacaagcct ccaagacggt     180

ccaggatgcg ctaagtagcg tgcaggagtc cgatatagct gtggtggcca ggggctggat     240

ggacaatcac ttcagatccc tgaaaggcta ctggagcaag tttactgaca agttcaccgg     300

cttctgggat tctaaccctg aggaccaacc aactccagct attgagtcgt gagacttctg     360

tgttgcagat gtgcctgttc ctccatcctg ctgcccccct ccaggcctgc caggtggccc     420

ctgaaggttg ctttaagggg aaagtatgtt ctcatgtctt cacccctccc tagatctcac     480

ctaaacatgc tgtccctaat aaagctggat aagaagctgc tgtt                    524


<210>  3
<211>  20
<212>  DNA
<213>  Artificial

<220>
<223>  ApoC3-1

<400>  3
tcttatccag ctttattagg                                                20


<210>  4
<211>  19
<212>  DNA
<213>  Artificial

<220>
<223>  ApoC3-2

<400>  4
ccggggctgc atggcacct                                                 19


<210>  5
<211>  16
<212>  DNA
<213>  Artificial

<220>
<223>  ApoC3-1-16

<400>  5
ttatccagct ttatta                                                    16


<210>  6
<211>  13
<212>  DNA
<213>  Artificial

<220>
<223>  ApoC3-1-BNA13

<400>  6
atccagcttt att                                                       13


<210>  7
<211>  13
<212>  DNA
<213>  Artificial

<220>
<223>  ApoC3-2-BNA13

<400>  7
ctgcatggca cct                                                       13

<210>  8
<211>  13
<212>  DNA
<213>  Artificial

<220>
<223>  ApoC3-3-BNA13

<400>  8
catggcacct acg                                                          13


<210>  9
<211>  13
<212>  DNA
<213>  Artificial

<220>
<223>  ApoC3-4-BNA13

<400>  9
tcgggcagat gcc                                                          13


<210>  10
<211>  13
<212>  DNA
<213>  Artificial

<220>
<223>  ApoC3-5-BNA13

<400>  10
tccatgtagc cct                                                          13


<210>  11
<211>  13
<212>  DNA
<213>  Artificial

<220>
<223>  ApoC3-6-BNA13

<400>  11
tcttggaggc ttg                                                          13


<210>  12
<211>  13
<212>  DNA
<213>  Artificial

<220>
<223>  ApoC3-7-BNA13

<400>  12
gctccagtag cct                                                          13


<210>  13
<211>  13

<212>  DNA
<213>  Artificial

<220>
<223>  ApoC3-8-BNA13

<400>  13
ttaaagcaac ctt                                                          13


<210>  14
<211>  13
<212>  DNA
<213>  Artificial

<220>
<223>  ApoC3-9-BNA13

<400>  14
gcagcttctt atc                                                          13


<210>  15
<211>  14
<212>  DNA
<213>  Artificial

<220>
<223>  ApoC3-46-BNA14

<400>  15
gctgcatggc acct                                                         14


<210>  16
<211>  14
<212>  DNA
<213>  Artificial

<220>
<223>  ApoC3-51-BNA14

<400>  16
ccggggctgc atgg                                                         14


<210>  17
<211>  13
<212>  DNA
<213>  Artificial

<220>
<223>  ApoC3-13-2-BNA

<400>  17
ctgcatggca cct                                                          13


<210>  18
<211>  25
<212>  DNA
<213>  Artificial

<220>
<223>  probe in ELISA for determining oligonucleotide in liver

<400>  18
gaatagcgat aataaagctg gataa                                          25


<210>  19
<211>  9
<212>  DNA
<213>  Artificial

<220>
<223>  template in ELISA for determining oligonucleotide in liver

<400>  19
tcgctattc                                                             9


<210>  20
<211>  14
<212>  DNA
<213>  Artificial

<220>
<223>  mApoC3-6-BNA(14)

<400>  20
tagggataaa actg                                                      14


<210>  21
<211>  14
<212>  DNA
<213>  Artificial

<220>
<223>  mApoC3-21-BNA(14)

<400>  21
gtagctagct gctt                                                      14


<210>  22
<211>  14
<212>  DNA
<213>  Artificial

<220>
<223>  mApoC3-36-BNA(14)

<400>  22
acctacgtac ctgg                                                      14


<210>  23
<211>  14
<212>  DNA
<213>  Artificial

<220>
<223>  mApoC3-66-BNA(14)

<400>  23
cacagtgagg agcg                                                          14


<210>  24
<211>  14
<212>  DNA
<213>  Artificial

<220>
<223>  mApoC3-81-BNA(14)

<400>  24
gagagccaag aggg                                                          14


<210>  25
<211>  14
<212>  DNA
<213>  Artificial

<220>
<223>  mApoC3-96-BNA(14)

<400>  25
tcgggcagat gcca                                                          14


<210>  26
<211>  14
<212>  DNA
<213>  Artificial

<220>
<223>  mApoC3-111-BNA(14)

<400>  26
ctctacctct tcag                                                          14


<210>  27
<211>  14
<212>  DNA
<213>  Artificial

<220>
<223>  mApoC3-126-BNA(14)

<400>  27
cagcagcaag gatc                                                          14


<210>  28
<211>  14
<212>  DNA
<213>  Artificial

<220>
<223>  mApoC3-141-BNA(14)

<400>  28
gccctgtaca gagc                                                          14

<210>  29
<211>  14
<212>  DNA
<213>  Artificial

<220>
<223>  mApoC3-156-BNA(14)

<400>  29
ggcttgttcc atgt                                                         14


<210>  30
<211>  14
<212>  DNA
<213>  Artificial

<220>
<223>  mApoC3-171-BNA(14)

<400>  30
ctggaccgtc ttgg                                                         14


<210>  31
<211>  14
<212>  DNA
<213>  Artificial

<220>
<223>  mApoC3-186-BNA(14)

<400>  31
gctacttagc gcat                                                         14


<210>  32
<211>  14
<212>  DNA
<213>  Artificial

<220>
<223>  mApoC3-201-BNA(14)

<400>  32
atcggactcc tgca                                                         14


<210>  33
<211>  14
<212>  DNA
<213>  Artificial

<220>
<223>  mApoC3-216-BNA(14)

<400>  33
ggccaccaca gcta                                                         14


<210>  34
<211>  14

<212>  DNA
<213>  Artificial

<220>
<223>  mApoC3-231-BNA(14)

<400>  34
gtccatccag cccc                                                          14


<210>  35
<211>  14
<212>  DNA
<213>  Artificial

<220>
<223>  mApoC3-246-BNA(14)

<400>  35
gaatctgaag tgat                                                          14


<210>  36
<211>  14
<212>  DNA
<213>  Artificial

<220>
<223>  mApoC3-261-BNA(14)

<400>  36
ccagtagcct ttca                                                          14


<210>  37
<211>  14
<212>  DNA
<213>  Artificial

<220>
<223>  mApoC3-276-BNA(14)

<400>  37
gtcagtaaac ttgc                                                          14


<210>  38
<211>  14
<212>  DNA
<213>  Artificial

<220>
<223>  mApoC3-291-BNA(14)

<400>  38
gaagccggtg aact                                                          14


<210>  39
<211>  14
<212>  DNA
<213>  Artificial

<220>
<223>  mApoC3-306-BNA(14)

<400>  39
agggttagaa tccc                                                          14


<210>  40
<211>  14
<212>  DNA
<213>  Artificial

<220>
<223>  mApoC3-321-BNA(14)

<400>  40
agttggttgg tcct                                                          14


<210>  41
<211>  14
<212>  DNA
<213>  Artificial

<220>
<223>  mApoC3-336-BNA(14)

<400>  41
cgactcaata gctg                                                          14


<210>  42
<211>  14
<212>  DNA
<213>  Artificial

<220>
<223>  mApoC3-351-BNA(14)

<400>  42
aacacagaag tctc                                                          14


<210>  43
<211>  14
<212>  DNA
<213>  Artificial

<220>
<223>  mApoC3-366-BNA(14)

<400>  43
aacaggcaca tctg                                                          14


<210>  44
<211>  14
<212>  DNA
<213>  Artificial

<220>
<223>  mApoC3-381-BNA(14)

<400>  44
gcagcaggat ggag                                                         14


<210>  45
<211>  14
<212>  DNA
<213>  Artificial

<220>
<223>  mApoC3-396-BNA(14)

<400>  45
caggcctgga gggg                                                         14


<210>  46
<211>  14
<212>  DNA
<213>  Artificial

<220>
<223>  mApoC3-411-BNA(14)

<400>  46
tcagggccca cctg                                                         14


<210>  47
<211>  14
<212>  DNA
<213>  Artificial

<220>
<223>  mApoC3-426-BNA(14)

<400>  47
cccttaaagc aacc                                                         14


<210>  48
<211>  14
<212>  DNA
<213>  Artificial

<220>
<223>  mApoC3-441-BNA(14)

<400>  48
tgagaacata cttt                                                         14


<210>  49
<211>  14
<212>  DNA
<213>  Artificial

<220>
<223>  mApoC3-456-BNA(14)

<400>  49
ggagggtga agac                                                          14

<210>  50
<211>  14
<212>  DNA
<213>  Artificial

<220>
<223>  mApoC3-471-BNA(14)

<400>  50
ttaggtgaga tcta                                                          14


<210>  51
<211>  14
<212>  DNA
<213>  Artificial

<220>
<223>  mApoC3-486-BNA(14)

<400>  51
ttagggacag catg                                                          14


<210>  52
<211>  14
<212>  DNA
<213>  Artificial

<220>
<223>  mApoC3-501-BNA(14)

<400>  52
tcttatccag cttt                                                          14


<210>  53
<211>  14
<212>  DNA
<213>  Artificial

<220>
<223>  mApoC3-511-BNA(14)

<400>  53
aacagcagct tctt                                                          14